# EUROPEAN PATENT SPECIFICATION

(11) **EP 0 758 656 B1**
(45) Date of publication and mention of the grant of the patent: **22.10.2003**
(21) Application number: 96112799.0
(22) Date of filing: 08.08.1996
(51) Int. Cl.: C07K 16/18, A61K 39/395

(54) **Immunoglobulin activated by mixing with a histamine component**
Immunoglobuline die durch Mischung mit einem Komponenten von Histamin aktiviert wurden
Immunoglobulins activés en mélangeant avec un composant de la histamine

(30) Priority: 11.08.1995 JP 22704595
(43) Date of publication of application: 19.02.1997
(73) Proprietor: NIPPON ZOKI PHARMACEUTICAL CO., LTD., Chuo-ku, Osaka (JP)
(72) Inventor: Yoshii, Haruo, c/o Institute of Bio-Active Science, Yashiro-cho, Katoh-gun, Hyogo (JP); Naiki, Mitsuru, Institute of Bio-Active Science, Yashiro-cho, Katoh-gun, Hyogo (JP); Fukata, Yuriko, Institute of Bio-Active Science, Yashiro-cho, Katoh-gun, Hyogo (JP)
(74) Representative: Hansen, Bernd, Dr. Dipl.-Chem.

(56) References cited:
- EP-A- 0 646 376
- WO-A-87/05221
- DATABASE WPI Section Ch, Week 8309 Derwent Publications Ltd., London, GB; Class A96, AN 83-21728K XP002032151 & RO 72 676 A (CANTACUZINO INST BUCURESTI) , 30 July 1982
- 9TH INTERNATIONAL CONGRESS OF IMMUNOLOGY. MEETING ABSTRACT NUMBER 1084, vol. 0, no. 0, 23 - 29 July 1995, SAN FRANCISCO, CALIFORINA, page 183 XP000672330 NAIKI M. ET AL.: "Rat gamma-globulin/histamine inhibits experimental allergic encephalomylitis (EAE) in Lewis rats"
- ARERUGI, vol. 44, no. 5, May 1995, JAPAN, pages 567-570, XP000653985 YOSHII H. ET AL.: "Inhibitory effect of hiatamine-added mouse gamma-globulin on eosinophil accumulation induced by allergen in BALB/c mice"

## Description

### Field of the Invention:

The present invention relates to a method of giving new pharmacological activity (such as eosinophilia-suppressive action, immunomodulating action, therapeutic action for autoimmune disease, antiinflammatory action, antiallergic action, etc. which are not inherent to immunoglobulin of natural type) to immunoglobulin; to activated immunoglobulin obtained by said method; and to a pharmaceutical composition containing said activated immunoglobulin.

### Background of the Invention:

When a foreign substance invades into a living organism, various reactions take place in the organism for removing the foreign substance. One of the reactions is the so-called immune reaction whereby a specific protein (antibody) corresponding to the foreign substance (antigen) is produced. The immune reaction is a vital reaction for defending the organism against the invasion of foreign substances such as pathogens and various other proteins, polysaccharides, etc. and its nature is based upon an antigen-antibody reaction in which antibody is bonded to antigen in a specific manner.

The main activity of an antibody is a binding activity which is specific to an antigen and, when the antigen is in a form of particles such as bacteria, agglutination reaction due to a formation of cross-linking of the antibody among the particles is induced while, when antigen has toxicity, enzymatic activity, etc., neutralization reaction due to a binding of antibody or hemolytic reaction, bacteriolytic reaction, immune adherence reaction, immunophagocytosis, etc: due to activation by binding of antigen-antibody complex with complement components in blood are induced whereupon an immune response reaction in the living organism against the invasion of the foreign substance is carried out.

Immunoglobulin is a generic name for antibody proteins and proteins, which are similar to the antibody proteins in terms of structures and functions, and is classified into five classes depending upon the properties. For example, IgG is a main component of immunoglobulin, is the highest in terms of both production amount and blood level, is produced continuously and has a long half life in blood. Therefore, it has been recognized as an antibody component which is important for maintaining the immunity continuously. On the other hand, IgM is produced in early stage even by stimulation of antigen in a small amount but its production is little and transient and it is thought to be the antibody playing a leading role in early protection. With respect to IgA, most of it is secreted to saliva, secretions from digestive organs and respiratory tracts and milk and is considered to play a main role for a direct protection against the infection through respiratory tracts and mouth from outside.

Based upon the antibody activity which is inherent to immunoglobulin as mentioned above, immunoglobulin preparations prepared from human serum have been used as pharmaceuticals. Preparations solely consisting of immunoglobulin of natural type prepared by mere purification and concentration of serum contain various antibodies against pathogens of various infectious diseases and products thereof and, therefore, they are admitted to use for prevention and improvement in symptoms of not only non- or hypoglobulinemia but also to viral diseases such as measles, hepatitis (type A) and poliomyelitis as well as to use together with antibiotics. Besides those, there are preparations of immunoglobulin of natural type prepared from a special serum which are used for special diseases such as tetanus and hepatitis of type B.

In order to make the intravenous injection of immunoglobulin of natural type possible, there is an immunoglobulin preparation of a treated type for intravenous injection in which enzymatic and chemical treatments and modifications are applied with the object of removing the agglutinated globulin molecules which are the cause of shock-like symptoms. For example, preparations which are treated with pepsin, plasmin, polyethylene glycol or ion exchange resin or treated at pH 4 and those in which immunoglobulin is alkylated or sulfonated are available. Like the preparations of immunoglobulin of natural type, pharmacological action of those preparations of processed type is also based upon the antibody action which is inherent to immunoglobulin.

RO 72676 describes the determination of antibodies reacting with e.g. histamine. Therein, a method is described comprising binding histamine to a column and contacting the column with a gamma globulin solution. Gamma globulin bound to histamine is obtained and purified by elution with 0.1 M glycocol HCl buffer, pH 2 resulting in a purified histamine specific gamma globulin fraction.

As such, the immunoglobulin preparations which are used at present are expected to show the therapeutic effect due to the above-mentioned physiological activity inherent to the antibody and there has been no report wherein globulin of natural type is processed to give a new pharmacological activity.

An object of the present invention is to offer a method of activating immunoglobulin for giving pharmacological activity such as eosinophilia-suppressive action, immunomodulating action, therapeutic action for autoimmune disease, antiinflammatory action and/or antiallergic action which is not inherently available in immunoglobulin of natural type. A further object is to provide said activated immunoglobulin. Yet an other object is to provide a pharmaceutical composition containing said activated immunoglobulin as an effective component.

### Detailed Description of the Invention:

The activated immunoglobulin according the present invention exhibits eosinophilia-suppressive action, immunomodulating action, therapeutic action for autoimmune disease, antiinflammatory action and/or antiallergic action.

It can be prepared by mixing immunoglobulin with a histamine component followed by removing the histamine component therefrom. Preferably, the immunoglobulin is obtained from a mammal. In the case of a pharmaceutical agent which is used for human being, it goes without saying that human immunoglobulin may be used as a material and such a human immunoglobulin can be obtained from serum or placenta plasma by common methods. In order to secure the safety as a pharmaceutical agent, the standards which are usually stipulated for plasma fraction preparations are to be satisfied. For example, when human plasma which is negative to HBs antigen, HCV antibody and HIV antibody is used and subjected to a heating treatment, danger of contamination of hepatitis virus and AIDS virus can be avoided. The heating treatment is commonly used for inactivation of virus and, for example, a liquidly heating treatment at 60°C for ten hours, an evaporating heating treatment at 60°C for ten hours, a drying heating treatment at 65°C for 96 hours, etc. are usually conducted for fractionated plasma preparations.

In the case of application to animals other than human being, immunoglobulin may be prepared from an animal other than human being depending upon the type of the animal to be treated. Free histamine and its pharmaceutically-acceptable salts such as hydrochloride, phosphate and picrate may be used as a histamine component.

When immunoglobulin is mixed with a histamine component and then the histamine component is removed therefrom, it is possible to activate the immunoglobulin of natural type which originally does not exhibit the pharmacological activity such as eosinophilia-suppressive action, immunomodulating action, therapeutic action for autoimmune disease, antiinflammatory action or antiallergic action. The mixing treatment may be carried out by dissolving immunoglobulin and histamine component in water or in aqueous solution such as a physiological saline solution and by stirring the solution at a temperature where the solution is not frozen and also the immunoglobulin is not thermally denatured (e.g. at room temperature) followed, if necessary, by allowing to stand and any special operation is not particularly necessary. With respect to a ratio of mixing the immunoglobulin with the histamine component, the histamine component amount by which the immunoglobulin is activated may be suitably set up. For . example, from 0.015 to 150µg or, preferably, from 0.075 to 75µg of histamine component (based upon the amount of histamine) may be used to 1 g of immunoglobulin although the present invention is not particularly limited to such a ratio.

After conducting the above-mentioned mixing treatment, the histamine component is removed therefrom to give the activated immunoglobulin of the present invention. Removal of the histamine component may be easily and conveniently carried out by common methods such as dialysis and gel filtration although any other method such as adsorption chromatography, ion exchange chromatography and affinity chromatography may be used as well so far as it can separate the activated immunoglobulin and histamine.

### Brief Explanation of Drawings:

- Fig. 1: is a graph showing the promoting action of the activated immunoglobulin of the present invention to anti-TNP antibody production.
- Fig. 2: is a graph showing the suppressing action of the activated immunoglobulin of the present invention to onset of experimental allergic cerebrospinal inflammation.

The method of manufacturing the activated immunoglobulin of the present invention will be further illustrated by way of the following examples.

### Examples:

Since mice and rats were used as experimental animals in the pharmacological test which will be given later, immunoglobulins of the animals which were used in the experiments (i.e. mouse immunoglobulin and rat immunoglobulin) were used instead of human immunoglobulin. Accordingly, a method of manufacturing activated mouse immunoglobulin will be given in the following working example as an example.

### Example 1. Manufacture of Activated Mouse Immunoglobulin

To 50 ml of a solution of mouse immunoglobulin (20 mg/ml) was added 50 µl of a solution of histamine dihydrochloride (0.25 mg/ml) and the mixture was gently stirred for two hours at room temperature. Then this solution was dialyzed at 4°C for three days using a dialysis membrane having a molecular cutoff from 12,000 to 14,000 to remove the histamine component whereupon a solution of activated mouse immunoglobulin of the present invention was obtained. To be more specific, 10-50 ml of the solution was dialyzed against two liters of physiological saline solution and the outer solution after the dialysis was changed for three times a day so that the histamine component was completely removed. After completion of the dialysis, this was dried by freezing and stored and, upon actual use, it was dissolved in water or in a physiological saline solution.

### Example 2. Measurement of Remaining Histamine Component

Histamine remaining in the activated mouse immunoglobulin of the present invention manufactured in the above example was measured by a radioimmunoassay (using Histamine Kit "Eiken"; trade name). This assay is a method in which standard histamine and a sample were acylated, incubated together with ¹²⁵I-labeled histamine and histamine antibody tube, then unreacted ¹²⁵I-labeled histamine is removed and radioactivity bonded to the histamine antibody tube is measured by a scintillation counter of a well type. The histamine concentration in the sample was determined from a standard curve prepared at the same time. Sensitivity of this measuring method is 0.2 nM and, in the above-mentioned activated mouse immunoglobulin of the present invention, no remaining histamine was detected.

Moreover, in an assay in which another histamine measuring system by an HPLC-fluorescence method, no remaining histamine was detected. Further, in the case where tritium-labeled histamine and immunoglobulin were mixed by the same manner as in Example 1 followed by dialyzing to remove histamine, the measured radioactivity in the final inner liquid after the dialysis was the same as that in the case of use of unlabeled histamine (control) and there was no trace of remaining histamine. As such, remaining histamine was not detected in any of the measuring methods and it was confirmed that, as a result of the above-mentioned dialysis treatment, histamine was substantially removed almost completely.

### Pharmacological Tests

### 1. Inhibitory Action to Hypereosinophilicity

### (1) Hypereosinophilic Model Induced by Ragweed Pollen Antigen

In accordance with the method of Kaneko et al. (Int. Archs Allergy Appl. Immunol., 96, 41-45 (1991)), a ragweed pollen extract (which was diluted to an extent of 1,000 times using a physiological saline solution) was hypodermically injected to female BALB/c mice of six to eight weeks age for sensitization at the dose of 0.1 ml on the initiation day and on the first day and 0.2 ml on the sixth, eighth and fourteenth days. On the twentieth day, 0.2 ml of a 1,000 times diluted ragweed antigen was intraperitoneally injected to the mice to induce the reaction. On the 24th hour after the induction, the peritoneal exudate cells were recovered and subjected to a Giemsa staining and the numbers of eosinophils etc. were counted. As a result, the numbers of the eosinopils were in peak after 24 hours from the induction.

The above-mentioned hypereosilophilic models were used for checking the action to the hypereosinophilicity by a hypodermic injection of the product of the present invention, i.e. a activated mouse immunoglobulin, at a dose of 150 mg/kg/day twice a week for three weeks until the induced day. Further test was conducted by administering the corresponding amount of natural type of mouse immunoglobulin (not-activated Immunoglobulin).

An example of the results is given in Table. 1. Incidentally, in the following test results, significant difference in the average values from the control was calculated by means of Student's t-test and is expressed with asterisks (*: p < 0.05; **: p < 0.01; ***: p < 0.001).

**Table 1**

| Tested Products | Numbers of Eosinophils Exudated to Peritoneum (x 10⁵ cells) |
|---|---|
| Not Sensitized | 0.19 ± 0.06 |
| Control | 5.56 ± 0.58 |
| Immunoglobulin | 5.63 ± 0.87 |
| Product of the Invention | 1.06 ± 0.25*** |

### 2. Immunomodulating Action

The immunomodulating action was measured using a production of antibody specific to trinitrophenyl (TNP) and also a delayed TNP-specific hypersensitivity (DTH) reaction as targets.

### (1) Preparation of Trinitrophenyl-Bonded Sheep Red Blood Cells (TNP-SRBC)

Trinitrobenzenesulfonic acid (TNBS) was dissovled in a physiological saline solution buffered with phosphoric acid to prepare a solution (40 mg/7.0 ml; pH 7.2) and then 1 ml of sheep red blood cell pellets was dropped thereinto with stirring. The mixture was allowed to stand at room temperature with stirring for several times under a light-shielding state and washed with a physiological saline solution three times. Then it was centrifuged at 3,000 rpm for five minutes and made into a solution of 5 x 10⁹/ml using a physiological saline solution.

### (2) Production of a TNP-Specific Antibody

TNP-SRBC (10⁹ cells) was intraperitoneally administered to male BALB/c mice of 6 to 8 weeks age and the anti-TNP antibody in serum was measured by an enzymatic immunoassay using a dinitrophenyl-bovine serum albumin (DNP-BSA). The result was that a potent antibody production of anti-TNP-IgM and anti-TNP-IgG was noted having a peak on 4th to 6th days and on 6th to 8th days.

### (3) TNP-Specific DTH Reaction

Using the same mice as in the case of the antibody production system of the above, the mice were sensitized with TNP-SRBC and, on the 14th day, 0.025 ml of TNBs (4.7 mg/ml) was injected into a right hind paw to induce a TNP-specific DTH reaction. After 24 hours from the induction, thickness of both paws was measured using a dial gauge and the difference in the thickness between the right and left paws was expressed as the intensity of the DTH reaction. The result was that, after 24 hours from the induction, a DTH reaction was clearly noted.

### (4) Measurement of the Action of the Tested Drugs

The above-mentioned test system was used for checking the actions of the activated mouse immunoglobulin (150 mg/kg/day), i.e. the product of the present invention, natural type of mouse immunoglobulin (150 mg/kg/day) and cyclosporin A (100 mg/kg/day) to the anti-TNP antibody production and to the TNP-specific DTH reaction by a hypodermic injection for four days from the sensitization with TNP-SRBC.

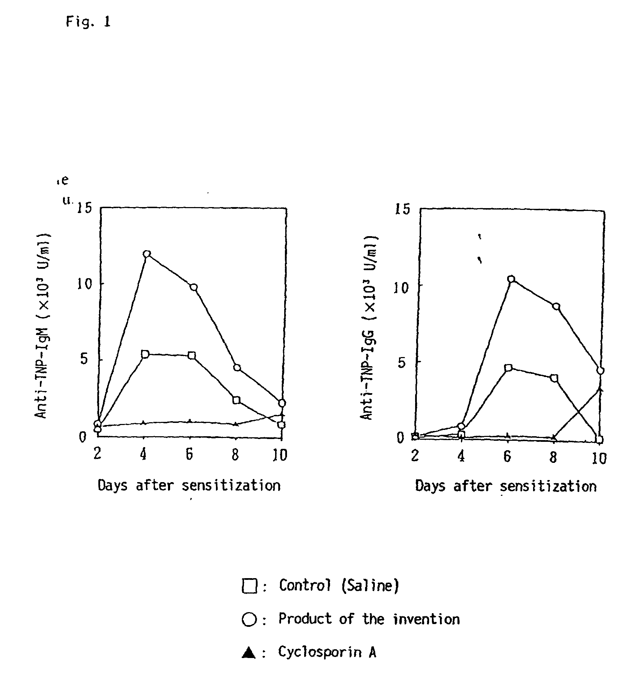

The result for the anti-TNP antibody production system is given in Fig. 1 while that for the TNP-specific DTH reaction system is given in Table 2.

**Table 2**

| Tested Drugs | Swelling In Paws (x 10⁻² mm) |
|---|---|
| Not Sensitized | 30.0 ± 3.4 |
| Control | 63.3 ± 4.0 |
| Immunoglobulin | 53.9 ± 5.0 |
| Product of the Invention | 35.8 ± 3.0*** |
| Cyclosporin | 35.8 ± 3.3*** |

### 3. Therapeutic action for autoimmune disease

Experimental allergic encephalomyelitis (EAE) has been used as a model for autoimmune disease, especially demyelinating disease such as multiple scleosis or postvaccinal encephalomyelitis. Passive EAE was induced by the established method (M. Naiki et al., Int. J. Immunopharmac., 13(2/3), 235-243 (1991) etc.). Synthetic peptide (MBP 68-84) corresponding to the encepalitogenic determinant of guinia pig meylin basic protein (MBP), residues 68-84, was dissolved in phosphate-buffered saline (0.2 mg/ml), and was emulsified in an equal volume of complete adjuvant (H37Ra) containing 2.5 mg/ml of heat inactivated tuberculosis. Female Lewis rats (body weight 160-170 g) were sensitized by inoculation with 0.1 ml of the emulsion in the left hind foot pad. After 12 days, spleen cells from the immunized rats were cultured in the presence of 2µg/ml of concanavalin A for 72 hours. After washing, 2 x 10⁷ cultured cells were injected intravenously into recipient rats to induce passive EAE.

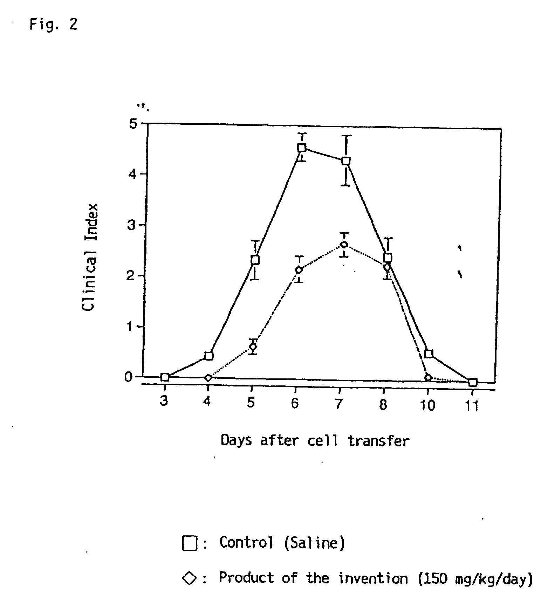

The rats were injected subcutaneously with the test drug in saline every other day for 8 days (4 times) after cell transfer and clinical indexes of the disease were assessed every day . A clinical index was used to grade animals on indexes of from 0 to 5 as follows; grade 0 = normal; grade 1 = inactive or tail weakness; grade 2 = weakness of hind legs or mild ataxia; grade 3 = hind legs paralysis of severe ataxia; grade 4 = severe hind legs paralysis; grade 5 = severe four legs paralysis or dying. An example of the clinical assessment of the therapeutic effects of the activated immunoglobulin of the present invention for EAE is shown in Fig. 2.

As shown in Table 1, the activated immunoglobulin of the present invention significantly inhibited the eosinophil exudation into peritoneum in them hypereosinophilic models induced by ragweed pollen antigen. It is also apparent--from the result of Fig. 1 that the activated immunoglobulin of the present invention showed remarkable promoting actions to IgM and IgG antibody productions. On the contrary, cyclosporin A which is an immunosuppressive agent markedly suppressed the production of both antibodies. However, as shown in Table 2, the activated immunoglobulin of the present invention showed an excellent suppressive action to delayed type hypersensitivity (DTH) like cyclosporin A.

As such, cyclosporin A which is a conventional immunosuppressive agent markedly suppressed both immunoreactions (IgM and IgG antibody productions and DTH reaction) while the activated immunoglobulin of the present invention showed promoting actions to antibody productions but exhibited suppressive action to the DTH reaction. Accordingly, it is apparent that the activated immunoglobulin of the present invention has an immunomodulating action which is clearly different from the conventional immunosuppressive agents.

It is also apparent from the result of Fig. 2 that the activated immunoglobulin of the present invention significantly suppressed the clinical symptoms as a result of onset of EAE (experimental allergic encephalomyelitis) which is an autoimmune disease model. On the contrary, in a group to which mouse immunoglobulin of natural type was administered, no EAE suppressive action was noted like the control.

The mixing ratio of immunoglobulin to histamine component in the manufacture of activated immunoglobulin of the present invention was investigated and, in the case of the activated immunoglobulin of the present invention manufactured in which the ratio of the histamine component to the immunoglobulin in Example 1 was made one-tenth, the product exhibited remarkable eosinophilia-suppressive and EAE-suppressive actions the same as the product of Example 1 but, when said ratio was changed to one-hundredth or ten-fold, the tendency of suppression was still noted although the suppressive action was not so strong enough. When oral administration was applied instead of subcutaneous injection, nearly the same pharmacological activity such as eosinophilia- and EAE-suppressive actions was noted.

In any of the above-mentioned pharmacological tests, no action was observed in common immunoglobulin which was not subjected to the activating treatment of the present invention. Accordingly, it is apparent that those pharmacological actions are the actions which are specifically given to immunoglobulin by the activating treatment of the present invention.

It is apparent from the results of the above-mentioned pharmacological tests that the activated immunoglobulin of the present invention prepared by an activating operation wherein immunoglobulin is mixed with histamine component followed by removing the histamine component therefrom exhibits useful pharmacological actions which are not available in immunoglobulin of natural type even though the product comprises a sole immunoglobulin component. Said excellent pharmacological actions are specific immunomodulating actions which are clearly different from those of conventional immunosuppressive agents such as cyclosporin A. Further, in addition to the fact that the activated immunoglobulin of the present invention has an eosinophilia suppressive action, it also shows a remarkable therapeutic effect for EAE which is an autoimmune disease model. Accordingly, it is useful as a pharmaceutical agent for the therapy of collagen diseases such as systemic lupus erythematodes and chronic rheumatoid arthritis; demyelinating diseases such as multiple sclerosis and postvaccinal encephalomyelitis; autoimmune diseases such as autoimmune hemolytic anemia, chronic thyroiditis and Hashimoto disease; and various immunodeficiency syndromes. Being based upon its suppressive action to eosinophilia, the activated immunoglobulin of the present invention can be also used as a therapeutic agent for eosinophilia caused by infectious diseases, parasitic diseases, diseases of respiratory organs, autoimmune diseases, malignant tumor, etc.

Eosinophil is known as an effecter cell which gathers to the stimulated area which is a cause of inflammation whereby inflammatory symptom is resulted. Accordingly, pharmaceutical agents which suppress an increase of eosinophils may be used as the agents for suppressing inflammation. In addition to the above-mentioned eosinophilia-suppressive action, the activated immunoglobulin of the present invention has been also confirmed to have a suppressive action to swelling in a DTH reaction and an improving effect to inflammatory autoimmune diseases (passive EAE) and is very highly useful as an excellent antiinflammatory agent as well. Further, eosinophil is deeply participated in an onset of allergic symptoms and the activated immunoglobulin of the present invention may be also used as a therapeutic and preventive agent for various kinds of allergic diseases such as bronchial asthma, allergic rhinitis, vasomotor rhinitis, urticaria, chronic eczema and atopic dermatitis.

The activated immunoglobulin of the present invention can be made into pharmaceutical preparations by a combination with a suitable pharmaceutical carriers or diluents. They can be made into various types of preparations by common methods and are able to be made into solid, semisolid, liquid or aerosol formulations for administrations by oral or parenteral means. In preparing the preparations, the activated immunoglobulin of the present invention can be used either solely or jointly together with other pharmaceutically-active components.

In the case of injections, it is preferable to be made into a pharmaceutical composition as an isotonic solution using distilled water for injection or physiological saline solution. In its manufacture, additives such as auxiliary solubilizers, isotonizing agents, stabilizers, buffers, preservatives, etc. may be used in addition to the activated immunoglobuline component. Examples of the applicable are citric acid, sodium benzoate, glycine, sodium sulfite, sodium bisulfite, sodium pyrosulfite, sodium thiosulfate, cysteine hydrochloride, phosphates, sodium ascorbate, sodium chloride, sodium bicarbonate, etc. Further, the product of the present invention may be prepared as an injectable preparation which is dissolved upon actual use. Thus, the immunoglobuline component may be prepared in a dry state or a solution is filled in vials or the like followed by a freeze-drying. In the manufacture of the dry preparation for injection, fillers such as glucose, mannitol and sorbitol may, if necessary, be added in additon to the above mentioned additives.

In the case of the preparations for oral administration, the activated immunoglobulin of the present invention as it is or together with commonly-used excipients such as a suitable additive (e.g. lactose, mannitol, corn starch, potato starch, etc.) is mixed with binders such as crystalline cellulose, cellulose derivatives, gum arabicum, corn starch, gelatin, etc., disintegrating agents such as corn starch, potato starch, carboxymethylcellulose potassium, etc., lubricating agents such as talc, magnesium stearate, etc. and others including bulking agents, moisturizing agents, buffers, preservatives, perfumes and the like to give tablets, diluted powders, granules or capsules.

It is also possible, depending upon the type of the disease or the condition of the patient to prepare the pharmaceutical preparations which are other than those which were mentioned already and are suitable for the therapy such as, for example, inhalations, aerosol preparations, ointments, collyriums, suppositories, etc.

The preferred dose of the activated immunoglobuline of the present invention may vary depending upon the type of the disease, the condition of the patient, age or sex of the patient, form of the preparation, method for the administration, term for the administration, etc. and, in order to achieve a desired effect, 1-300 mg, preferably 5-150 mg may be usually given to common adults once or several times a week by hypodermic injection, although the present invention is not particularly limited to such dosage.

## Claims

1. Medicament containing an activated immunoglobulin having eosinophilia-suppressive action, immunomodulating action, therapeutic action for autoimmune disease, antiinflammatory action and/or antiallergic action wherein the activated immunoglobulin is prepared by mixing immunoglobulin with a histamine component, and removing histamine therefrom again.

2. Medicament according to claim 1 wherein the histamine component is free histamine or a pharmaceutically acceptable salt thereof selected from hydrochloride, phosphate and picrate.

3. Medicament according to claim 1 wherein the mixing is carried out by dissolving immunoglobulin and the histamine component in water or in an aqueous solution, stirring the solution at a temperature where the solution is not frozen and also the immunoglobulin is not thermally denatured, and optionally followed by allowing to stand.

4. Medicament according to any of claims 1 to 3 wherein the mixing ratio of the immunoglobulin with the histamine component is set from 1 g immunoglobulin to 0.015 to 150 µg based on the free histamine in the histamine component.

5. Medicament according to claim 1 wherein the histamine component is removed by a separation treatment selected from dialysis, gel filtration, adsorption chromatography, ion exchange chromatography, and affinity chromatography.

6. Medicament according to any one of claims 1 to 5 wherein the activated immunoglobulin is obtained from serum or placenta plasma. .

7. Medicament according to any one of claims 1 to 6 wherein the activated immunoglobulin is obtained from a mammal.

8. Medicament according to claim 7 wherein the activated immunoglobulin is obtained from a human.

9. Medicament according to any one of claims 1 to 8 wherein the activated immunoglobulin has been virus-inactivated.

10. The medicament according to any one of claims 1 to 9, which is an eosinophilia-suppressing agent, an immunomodulating agent, a therapeutic agent for autoimmune disease, an inflammatory agent, and/or an antiallergic agent.

11. The medicament according to any one of claims 1 to 6 formulated as an injectable or an orally administerable preparation.

12. Use of an activated immunoglobulin wherein the activated immunoglobulin is prepared by mixing immunoglobulin with a histamine component, and removing histamine therefrom again, for the preparation of a medicament useful as an eosinophilia-suppressing agent, an immunomodulating agent, a therapeutic agent for autoimmune disease, an inflammatory agent, and/or an antiallergic agent.

## Patentansprüche

1. Medikament, enthaltend aktiviertes Immunglobulin mit Eosinophilie-unterdrückender Wirkung, immunmodulierender Wirkung, therapeutischer Wirkung bei Autoimmunerkrankung, antiinflammatorischer Wirkung und/oder antiallergischer Wirkung, wobei das aktivierte Immunglobulin hergestellt wird durch Mischen des Immunglobulins mit einer Histaminkomponente und anschliessendem Entfernen des Histamins.

2. Medikament gemäss Anspruch 1, wobei die Histaminkomponente freies Histamin ist oder ein pharmazeutisch annehmbares Salz davon, ausgewählt aus dem Hydrochlorid, Phosphat und Picrat.

3. Medikament gemäss Anspruch 1, wobei das Mischen durchgeführt wird durch Lösen des Immunglobulins und der Histaminkomponente in Wasser oder einer wässrigen Lösung, Rühren der Lösung bei einer Temperatur, bei der die Lösung nicht gefroren ist und bei der das Immunglobulin nicht thermisch denaturiert ist, und gegebenenfalls gefolgt von Stehenlassen.

4. Medikament gemäss einem der Ansprüche 1 bis 3, wobei das Mischungsverhältnis des Immunglobulins mit der Histaminkomponente eingestellt ist auf 1 g Immunglobulin zu 0,015 bis 150 µg freies Histamin in der Histaminkomponente.

5. Medikament gemäss Anspruch 1, wobei die Histaminkomponente entfernt wird durch Trennungsverfahren, ausgewählt aus Dialyse, Gelfiltration, Absorptionschromatographie, Ionenaustauschchromatographie und Affinitätschromatografie.

6. Medikament gemäss einem der Ansprüche 1 bis 5, wobei das aktivierte Immunglobulin erhalten wird aus Serum oder Plazentaplasma.

7. Medikament gemäss einem der Ansprüche 1 bis 6, wobei das aktivierte Immunglobulin aus Säugetieren erhalten wird.

8. Medikament gemäss Anspruch 7, wobei das aktivierte Immunglobulin aus Menschen erhalten wird.

9. Medikament gemäss einem der Ansprüche 1 bis 8, wobei das aktivierte Immunglobulin virusinaktiviert wurde.

10. Medikament gemäss einem der Ansprüche 1 bis 9, das ein Eosinophilie-unterdrückendes Mittel, ein immunmodulierendes Mittel, ein therapeutisches Mittel bei Autoimmunerkrankung, ein inflammatorisches Mittel und/oder ein antiallergisches Mittel ist.

11. Medikament gemäss einem der Ansprüche 1 bis 6, formuliert als injizierbare oder oral verabreichbare Präparation.

12. Verwendung von aktiviertem Immunglobulin, wobei das aktivierte Immunglobulin hergestellt ist durch Mischen des Immunglobulins mit einer Histaminkomponente und anschliessendes Entfernen des Histamins zur Herstellung eines Medikaments, das als Eosinophilie-unterdrückendes Mittel, immunmodulierendes Mittel, therapeutisches Mittel bei Autoimmunerkrankung, inflammatorisches Mittel und/oder antiallergisches Mittel nützlich ist.

## Revendications

1. Médicament comprenant une immunoglobuline activée ayant une action suppressive de l'éosinophilie, une action immunomodulante, une action thérapeutique pour une maladie autoimmune, un action anti-inflammatoire et/ou une action antiallergique dans lequel l'immunoglobuline activée est préparée en mélangeant l'immunoglobuline avec un composant de l'histamine, et en enlevant à nouveau l'histamine de celle-ci.

2. Médicament selon la revendication 1, dans lequel le composant de l'histamine est de l'histamine libre ou un sel de celle-ci acceptable du point de vue pharmaceutique choisi parmi un chlorhydrate, un phosphate et un picrate.

3. Médicament selon la revendication 1, dans lequel le mélange est effectué en dissolvant une immunoglobuline et le composant de l'histamine dans de l'eau ou dans une solution aqueuse, en agitant la solution à une température à laquelle la solution n'est pas gelée et aussi où l'immunoglobuline n'est pas dénaturée de façon thermique, et optionnellement suivi en la laissant au repos.

4. Médicament selon l'une quelconque des revendications 1 à 3, dans lequel le rapport de mélange entre l'immunoglobuline et le composant de l'histamine est fixé à 1 g d'immunoglobuline pour 0,015 à 150 mg basé sur l'histamine libre dans le composant de l'histamine.

5. Médicament selon la revendication 1, dans lequel le composant de l'histamine est enlevé par un traitement de séparation choisi parmi une dialyse, une filtration sur gel, une chromatographie par adsorption, une chromatographie par échange d'ion, et une chromatographie par affinité.

6. Médicament selon l'une quelconque des revendications 1 à 5, dans lequel l'immunoglobuline activée est obtenue à partir d'un sérum ou d'un plasma de placenta.

7. Médicament selon l'une quelconque des revendications 1 à 6, dans lequel l'immunoglobuline activée est obtenue à partir d'un mammifère.

8. Médicament selon la revendication 7, dans lequel l'immunoglobuline activée est obtenue à partir d'un humain.

9. Médicament selon l'une quelconque des revendications 1 à 8, dans lequel les virus dans l'immunoglobuline activée ont été inactivés.

10. Médicament selon l'une quelconque des revendications 1 à 9, qui est un agent suppressif de l'éosinophilie, un agent immunomodulant, un agent thérapeutique pour une maladie autoimmune, un agent anti-inflammatoire et/ou un agent antiallergique.

11. Médicament selon l'une quelconque des revendications 1 à 6 élaboré sous la forme d'une préparation injectable ou administrable oralement.

12. Utilisation d'une immunoglobuline activée, dans laquelle l'immunoglobuline activée est préparée en mélangeant l'immunoglobuline avec un composant de l'histamine, et en enlevant à nouveau l'histamine de celle-ci, pour la préparation d'un médicament utile comme un agent suppressif de l'éosinophilie, un agent immunomodulant, un agent thérapeutique pour maladie autoimmune, un agent anti-inflammatoire et/ou un agent antiallergique.
